# EUROPÄISCHE PATENTSCHRIFT

(11) **EP 0 392 344 B1**
(45) Veröffentlichungstag und Bekanntmachung des Hinweises auf die Patenterteilung: **28.07.1993**
(21) Anmeldenummer: 90106495.6
(22) Anmeldetag: 05.04.1990
(51) Int. Cl.: C07C 311/39, A61K 31/18

(54) **Naphthylalkylamino-substituierte Sulfamoylbenzoesäurederivate, Verfahren zu ihrer Herstellung und ihre Verwendung als Heilmittel**
Naphthyl-alkyl-amino substituted sulfamoyl benzoic acid derivatives, process for their production and their use as pharmaceutical preparations
Dérivés de l'acide sulfamoylbenzoiques substitués par un groupe naphtylalcoylamino, procédé pour leur préparation et leur utilisation comme préparations pharmaceutiques

(30) Priorität: 08.04.1989 DE 3911549
(43) Veröffentlichungstag der Anmeldung: 17.10.1990
(73) Patentinhaber: HOECHST AKTIENGESELLSCHAFT, 65926 Frankfurt am Main (DE)
(72) Erfinder: Ellory, John Clive Prof., Oxford OX1 3PT (GB); Englert, Heinrich Christian Dr., D-6238 Hofheim am Taunus (DE); Lang, Hans-Jochen Dr., D-6238 Hofheim am Taunus (DE); Mania, Dieter Dr., D-6240 Königstein/Taunus (DE); Merkel, Wulf Dr., D-3550 Marburg (DE)

(56) Entgegenhaltungen:
- DE-A- 2 654 795
- GB-A- 1 434 405
- DIURETICS, Chemistry, Pharmacology and Medicine, 1983, JOHN WILEY & SONS, New York, Chichester, Brisbane, Toronto, Singapore; R. C. ALLEN: "Diuretic Activity of 4-Substituted-3-amino-5-sulfamoylbenzoic Acids", Seite 162

## Beschreibung

Die Erfindung betrifft Naphthylalkylamino-substituierte Sulfamoylbenzoesäurederivate sowie Verfahren zu ihrer Herstellung und ihre Verwendung als Heilmittel gegen Sichelzellenanämie. In den erfindungsgemäßen Verbindungen I 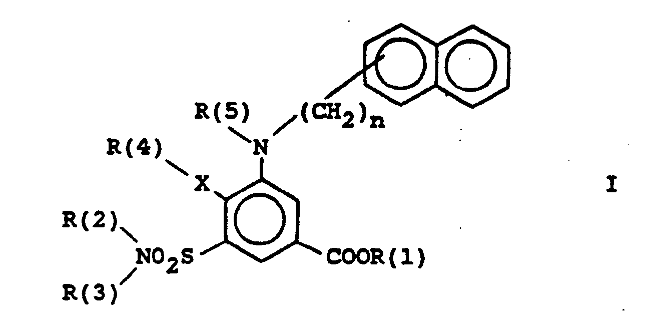
haben die folgenden Substituenten folgende Bedeutungen:
- R(1): Wasserstoff, (C₁-C₄)-Alkyl sowie Na , K , NH₄ , Ca , Mg ,
- R(2) und R(3): Wasserstoff, gleiche oder verschiedene (C₁-C₄)-Alkylgruppen, die auch cyclisch miteinander verbunden sein können,
- X: Sauerstoff, Schwefel, SO, SO₂, NR(6) [mit R(6) = Wasserstoff oder (C₁-C₂)-Alkyl ],
CH₂, CO, oder eine Bindung,
- R(4): Phenyl, Thienyl, die unsubstituiert sind oder durch 1 bis 2 Substituenten substituiert, welche aus der Gruppe F, Cl, Br, (C₁-C₂)-Alkyl, (C₁-C₂)-Alkoxy, Methylendioxy, S-(C₁-C₂)-Alkyl ausgewählt sind,
- R(5): Wasserstoff, (C₁-C₄)-Alkyl,
- n: 1, 2, 3 oder 4,

wobei das Naphthylsystem unsubstituiert ist oder wie der Phenylrest von R(4) substituiert ist,
und wobei X und R(4) gemeinsam auch Cl bedeuten können.

Bevorzugt sind Verbindungen I, in denen bedeuten:
- R(1): Wasserstoff, Na, K, Ca, Mg,
- R(2), R(3), R(5): Wasserstoff,
- X: Sauerstoff oder eine Bindung,
- R(4): Phenyl, Thienyl,
- n=: 2.

Steht R(1) für Na, K, Ca, Mg, so handelt es sich um Salze der Verbindungen I.

Die Erfindung betrifft weiterhin Verfahren zum Herstellen von Verbindungen I. Dabei setzt man
a) Verbindungen II 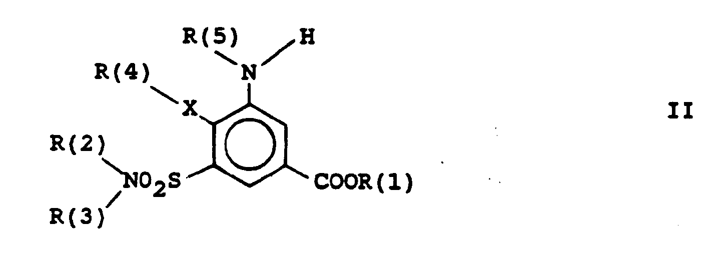 in welchen R(1) bis R(5) und X die angegebenen Bedeutungen haben, in an sich bekannter Weise,
   um mit Verbindungen III 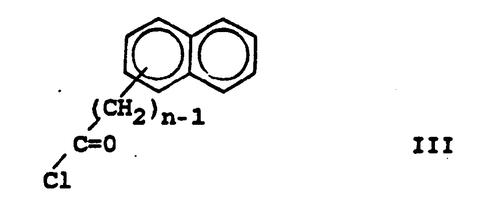 und reduziert die entstandenen Verbindungen V 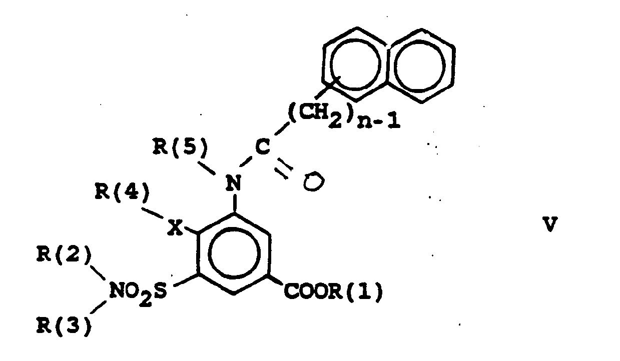 in an sich bekannter Weise, zu Verbindungen I,
   oder
b) man setzt Verbindungen II 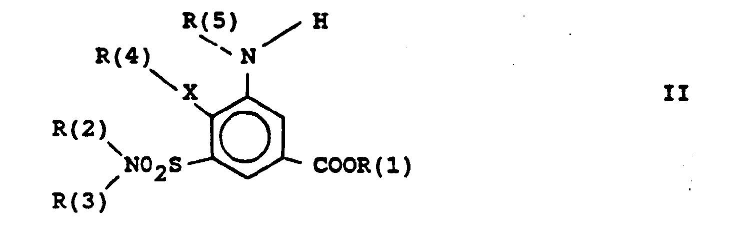 in an sich bekannter Weise um mit Verbindungen IV

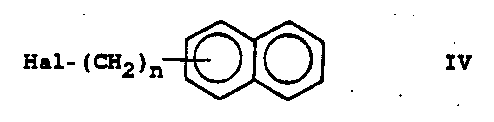

Während dieser Herstellungsverfahren können die beiden Gruppen R(2) und R(3) gemeinsam auch die bei solchen Reaktionen üblichen Schutzgruppen darstellen, deren Verwendung und nachfolgende Abspaltung beispielsweise in der deutschen Patentschrift 24 61 601 beschrieben ist.

Die erfindungsgemäßen Verbindungen I eignen sich als Heilmittel gegen Sichelzellenanämie, indem sie das Schrumpfen der Erythrocyten verzögern und somit einer sichelartigen Verformung der Erythrocyten entgegen wirken, die häufig im desoxygenierten Zustand erfolgt und, wie bekannt, den Blutdurchfluß durch die Kapillaren behindert. Die Verbindungen I inhibieren das KCl-Symport-System an Erythrocyten, das - wie bekannt - bei einer Volumenverringerung der Erythrocyten aktiviert wird.

Die erfindungsgemäßen Verbindungen I eignen sich für die Behandlung einer akuten Sichelzellenanämie oder auch für eine präventive Therapie.

Dabei werden, bezogen auf einen Menschen des Gewichtes von 75 kg, als Gesamttagesdosis mindestens 0,1 mg/kg Körpergewicht, vorzugsweise 0,5 mg, insbesondere 1 mg/kg Körpergewicht eingesetzt, die maximale Tagesdosis beträgt 10 mg, vorzugsweise 5 mg, insbesondere 1 mg/kg Körpergewicht.

Diese Tagesdosen können oral, intravenös oder rektal appliziert werden, und zwar in einer oder in mehreren Dosen.

Verbindungen ähnlich den erfindungsgemäßen Verbindungen I, jedoch anstelle der Naphthylgruppierung mit Phenyl sind bekannt aus DIURETICS, Chemistry, Pharmacology, and Medicine, JOHN WILEY & SONS, New York, Chichester, Brisbane, Toronto, Singapore, 1983, Seite 162, Table 3.46. Sie sind jedoch nur als Diuretika beschrieben, von einer anderen Wirksamkeit ist keine Rede.

Die erfindungsgemäßen Verbindungen I besitzen keine nennenswerte diuretische Wirksamkeit.

Die Herstellung der Ausgangsverbindungen II erfolgt nach dem Verfahren, wie es in der deutschen Patentschrift 26 54 795 beschrieben ist, oder auch nach dem Verfahren der deutschen Patentschrift 24 61 601.

### Beispiel 1

### 3-(Naphth-2'-ylethylamino)-4-phenoxy-5-sulfamoyl-benzoesäure

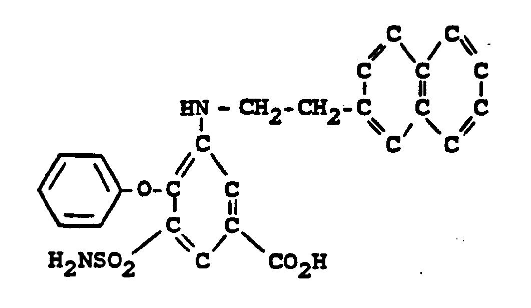

In eine Suspension von 3 g des 3-(Naphth-2'-ylethylamino)-4-phenoxy-5-N,N-dimethylaminomethylenaminosulfonylbenzoesäuremethylester in 40 ml Ethanol werden 3 g NaOH-Plätzchen eingetragen und dann wird bis zur klaren Lösung bei 60 bis 70°C gerührt. Nach Verdünnen mit 40 ml Wasser wird eine weitere halbe Stunde bei 70°C gerührt. Nach dem Abkühlen wird bei ∼ 0°C die Lösung mit überschüssiger konz. HCl angesäuert. Der Niederschlag wird abfiltriert, mehrfach mit kaltem Wasser gewaschen und getrocknet.
Kristalle vom Schmelzpunkt 270°C.

### Herstellung der Ausgangsverbindungen

### a) 3-(Naphth-2'-ylacetamido)-4-phenoxy-5-N,N-dimethylaminomethylenaminosulfonyl-benzoesäuremethylester

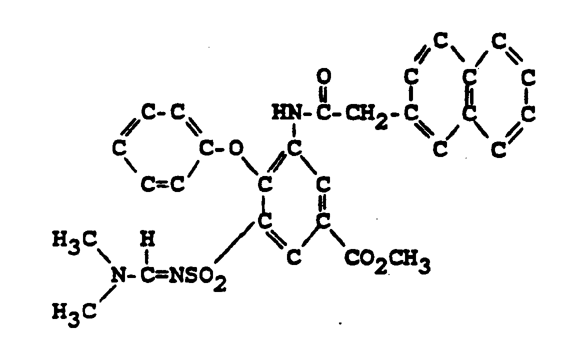

Zu einer Lösung von 20 g (0,053 mol) 3-Amino-4-phenoxy-5-N,N-dimethylaminomethylenaminosulfonylbenzoesäuremethylester in 200 ml Dioxan und 7,3 ml (0,09 Mol) Pyridin wird bei 80°C eine Lösung von 18,5 g (0,09 Mol) 2-Naphthyl-acetylchlorid in 65 ml Aceton zugetropft. Nach zweistündigem Rühren bei 80°C wird abgekühlt, die Lösung in Eiswasser eingetragen und mit Methylenchlorid extrahiert. Nach Trocknen über Na₂SO₄ wird die Methylenchloridlösung im Vakuum eingedampft und der Rückstand aus Methanol umkristallisiert.
Kristalle von Schmelzpunkt 127 - 128°C.

### b) 3-(Naphth-2'-ylethylamino)-4-phenoxy-5-N,N-dimethylaminomethylenaminosulfonylbenzoesäuremethylester

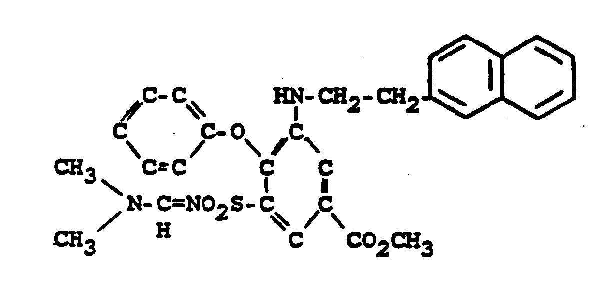

Zu einer Lösung von 11,5 g (0,021 Mol) 3-(Naphth-2'-ylacetamido)-4-phenoxy-5-N,N-dimethylaminomethylenaminosulfonyl-benzoesäuremethylester in einer Mischung aus 80 ml Diglyme und 4,5 ml (0,035 Mol) BF₃-Etherat wird eine Lösung von 1 g (0,026 Mol) NaBH₄ in 52 ml Diglyme zugetropft. Nach mehrstündigem Rühren bei 20°C - der Fortgang der Reaktion wird dünnschichtchromatographisch verfolgt (Kieselgel, Essigester/Petrolether 4 : 1) - wird die Reaktionslösung in Eiswasser eingetragen. Der Niederschlag wird abgesaugt, getrocknet und mehrfach aus Ethanol/DMF umkristallisiert.
Kristalle vom Schmelzpunkt 159°C.

### Beispiel 2

### 3-(Naphth-1'-ylethylamino)-4-phenoxy-5-sulfamoyl-benzoesäure

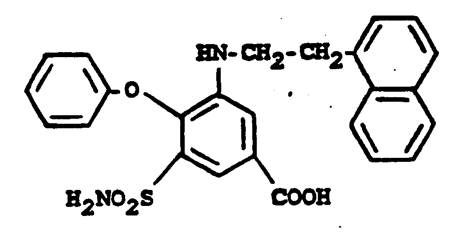

Analog Beispiel 1.
Weiße Kristalle vom Schmelzpunkt: 233 - 235°C.

### Beispiel 3

### 3-(Naphth-2'-ylethylamino)-4-(thien-2'-yl)-5-sulfamoyl-benzoesäure

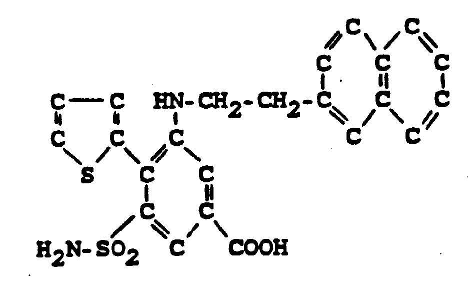

Analog Beispiel 1.
Schmelzpunkt: 208 - 209°C.

### Beispiel 4

### 4-Chlor-3-(naphth-2'-ylethylamino)-5-sulfamoyl-benzoesäure

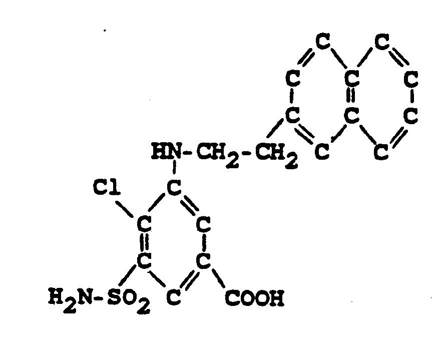

Analog Beispiel 1.
Schmelzpunkt: 230°C.

### Beispiel 5

### 3-(Naphth-2'-ylethylamino)-4-phenyl-5-sulfamoylbenzoesäure

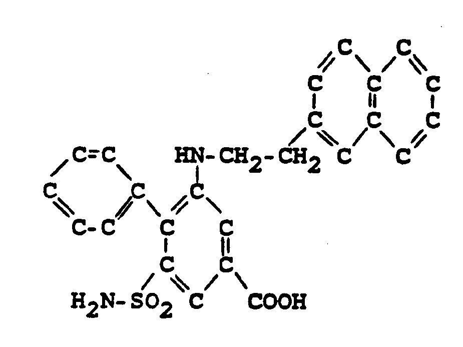

Analog Beispiel 1.
Schmelzpunkt: 223 - 224°C.

### Beispiel 6

### 3-(Naphth-1'-ylethylamino)-4-phenyl-5-sulfamoylbenzoesäure

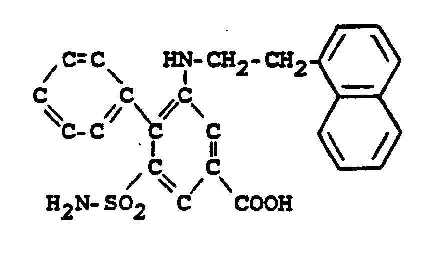

Analog Beispiel 1.
Schmelzpunkt: 217 - 218°C.

### Beispiel 7

### 3-(Naphth-1'-ylethylamino)-4-(thien-2'-yl)-5-sulfamoylbenzoesäure

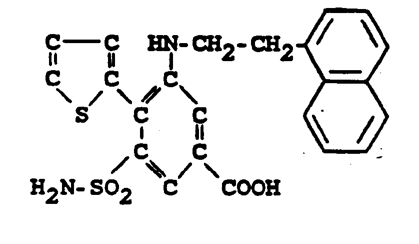

Analog Beispiel 1.
Schmelzpunkt: 192°C.

### Beispiel 8

### 4-Chlor-3-(naphth-1'-ylethylamino)-5-sulfamoylbenzoesäure

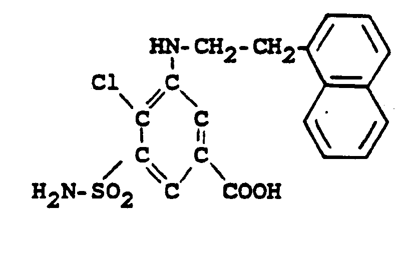

Analog Beispiel 1.
Schmelzpunkt: 151 - 152°C.

## Patentansprüche

1. Verbindung I 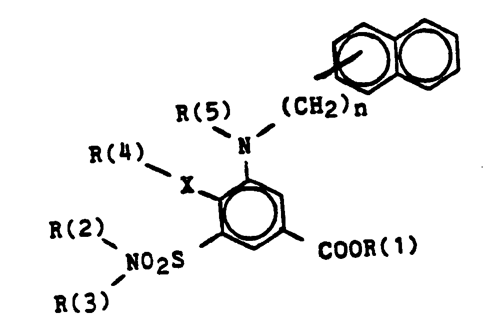 in der die folgenden Substituenten folgende Bedeutung haben:
R(1) Wasserstoff, (C₁-C₄)-Alkyl sowie Na, K, NH₄, Ca, Mg,
R(2) und R(3) Wasserstoff, gleiche oder verschiedene (C₁-C₄)-Alkylgruppen, die auch cyclisch miteinander verbunden sein können,
X Sauerstoff, Schwefel, SO, SO₂, NR(6) (mit R(6) = Wasserstoff oder (C₁-C₂-Alkyl) ,
CH₂, CO, oder eine Bindung,
R(4) Phenyl, Thienyl, die unsubstituiert sind oder durch 1 bis 2 Substituenten substituiert, welche aus der Gruppe F, Cl, Br, (C₁-C₂)-Alkyl, (C₁-C₂)-Alkoxy, Methylendioxy, S-(C₁-C₂)-Alkyl ausgewählt sind,
R(5) Wasserstoff, (C₁-C₄)-Alkyl,
n 1, 2, 3 oder 4,
wobei das Naphthylsystem unsubstituiert ist oder wie der Phenylrest von R(4) substituiert ist,
und wobei X und R(4) gemeinsam auch Cl bedeuten können.

2. Verbindung nach Anspruch 1, dadurch gekennzeichnet, daß folgenden Substituenten folgende Bedeutung haben:
R(1) Wasserstoff, Na, K, Ca, Mg,
R(2), R(3), R(5) Wasserstoff,
X Sauerstoff oder eine Bindung,
R(4) Phenyl, Thienyl,
n= 2.

3. Verfahren zum Herstellen einer Verbindung I nach Anspruch 1, dadurch gekennzeichnet, daß man
a) Verbindungen II 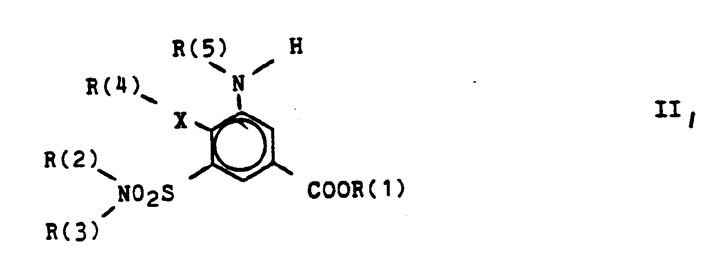 in welchen R(1) bis R(5) und X die angegebenen Bedeutungen haben, in an sich bekannter Weise,
mit Verbindungen III 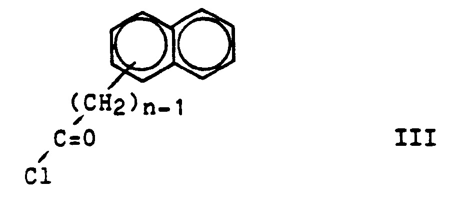 umsetzt
und die entstandenen Verbindungen V 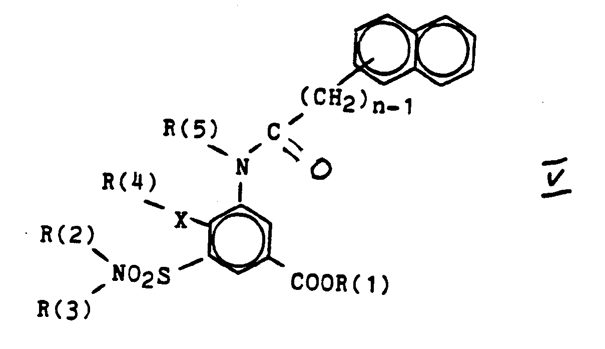 in an sich bekannter Weise, zu Verbindungen I reduziert,
oder daß
b) man Verbindungen II 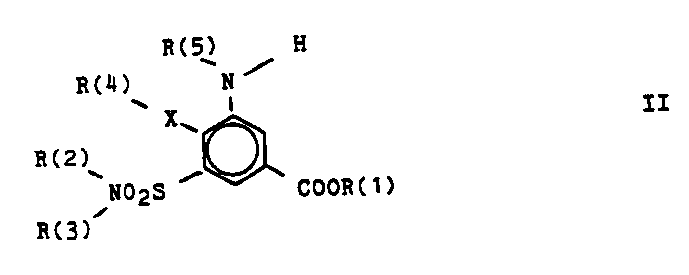 in an sich bekannter Weise mit Verbindungen IV 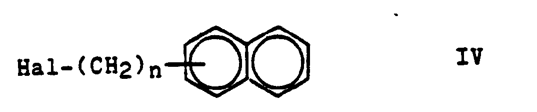 umsetzt.

4. Heilmittel zur Behandlung der Sichelzellenanämie bestehend aus einer wirksamen Menge einer Verbindung I nach Anspruch 1 und den üblichen Zusatzstoffen.

5. Verwendung einer Verbindung I nach Anspruch 1 zur Herstellung eines Medikaments zur Behandlung der Sichelzellenanämie.

6. Eine Verbindung I nach Anspruch 1 zur Verwendung in einem verfahren zum Behandeln der Sichelzellenanämie.

## Claims

1. A compound I 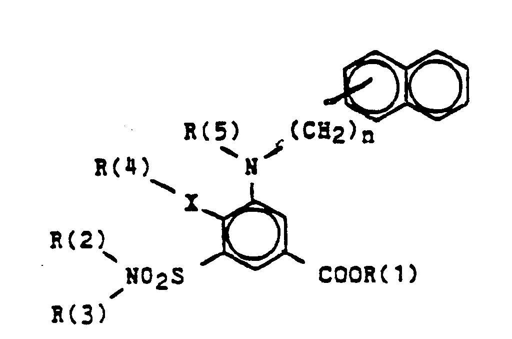 in which the following substituents have the following meaning:
R(1) hydrogen, (C₁-C₄)-alkyl and Na, K, NH₄, Ca, Mg,
R(2) and R(3) hydrogen, identical or different (C₁-C₄)- alkyl groups which can also be linked together in a ring,
X oxygen, sulfur, SO, SO₂, NR(6) (with R(6) = hydrogen or (C₁-C₂)-alkyl), CH₂, CO, or a bond,
R(4) phenyl, thienyl, which are unsubstituted or substituted by 1 to 2 substituents which are selected from the group comprising F, CI, Br, (C₁-C₂)-alkyl, (C₁-C₂)-alkoxy, methylenedioxy, S-(C₁-C₂)-alkyl,
R(5) hydrogen, (C₁-C₄)-alkyl,
n 1, 2, 3 or 4,
where the naphthyl system is unsubstituted or substituted like the phenyl radical of R(4), and where X and R(4) together can also be Cl.

2. A compound as claimed in claim 1, wherein the following substituents have the following meaning:
R(1) hydrogen, Na, K, Ca, Mg
R(2), R(3), R(5) hydrogen,
X oxygen or a bond,
R(4) phenyl, thienyl,
n = 2.

3. A process for the preparation of a compound I as claimed in claim 1, which comprises
a) reacting compounds II 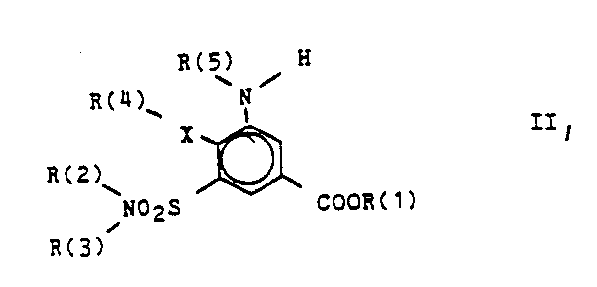 in which R(1) to R(5) and X have the stated mean- ings, in a manner known per se, with compounds III 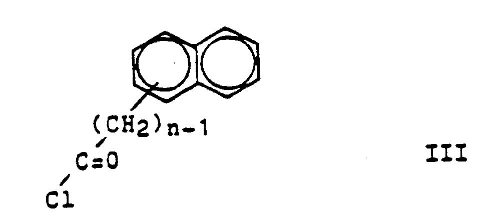 and reducing the resulting compounds V 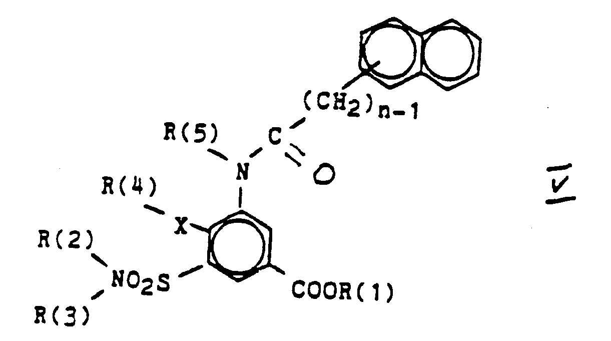 in a manner known per se, to compounds I, or
b) reacting compounds II 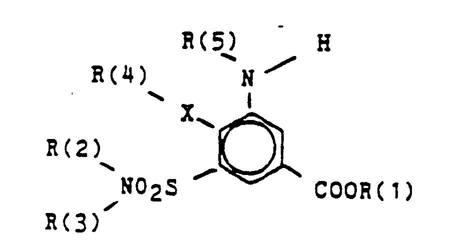 in a manner known per se, with compounds IV

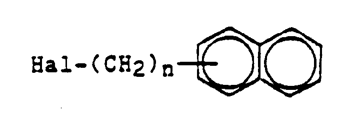

4. A medicine for the treatment of sickle-cell anemia composed of an effective amount of a compound I as claimed in claim 1 and of the customary additives.

5. The use of a compound I as claimed in claim 1 for the preparation of a medicament for the treatment of sickle-cell anemia.

6. A compound I as claimed in claim 1 for use in a method for the treatment of sickle-cell anemia.

## Revendications

1. Composé I 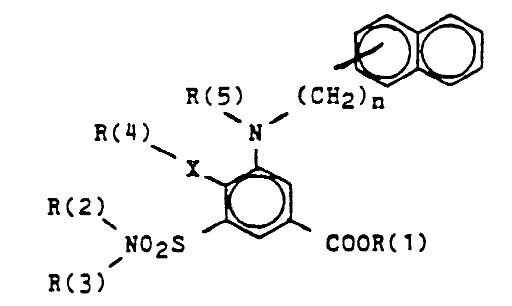 dans lequel les substituants suivants ont la signification suivante :
R(1) : hydrogène, alkyle en C₁ à C₄ ainsi que Na, K, NH₄, Ca, Mg,
R(2) et R(3) : hydrogène, groupes alkyle en C₁ à C₄, identiques ou différents, pouvant également être liés l'un à l'autre de manière à former un cycle,
X : oxygène, soufre, SO, SO₂, NR(6), (avec R(6) = hydrogène ou alkyle en C₁ à C₂),
CH₂, CO ou une liaison,
R(4) : phényle, thiényle, non substitués ou bien substitués par 1 à 2 substituants choisis dans l'ensemble constitué par F, Cl, Br, alkyle en C₁ à C₂, alcoxy en C₁ à C₂, méthylènedioxy, S-(alkyle en C₁ à C₂),
R(5) : hydrogène, alkyle en C₁ à C₄,
n : 1, 2, 3 ou 4,
où le système naphtylique est non substitué ou substitué comme le radical phényle de R(4),
et où X et R(4) peuvent aussi, ensemble, représenter Cl.

2. Composé selon la revendication 1, caractérisé en ce que les substituants suivants ont la signification suivante :
R(1) : hydrogène, Na, K, Ca, Mg,
R(2), R(3), R(5) : hydrogène,
X : oxygène ou une liaison,
R(4) : phényle, thiényle,
n = 2.

3. Procédé pour préparer un composé I conforme à la revendication 1, caractérisé en ce que l'on fait réagir :
a) des composés II 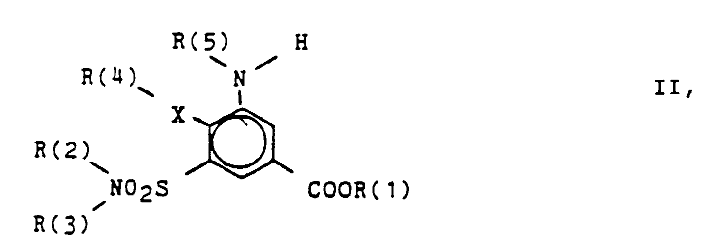 dans lesquels R(1) à R(5) et X présentent les significations mentionnées, de manière connue en elle-même,
avec des composés III 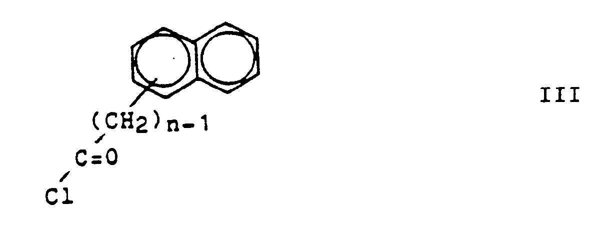 et on réduit les composés V résultants 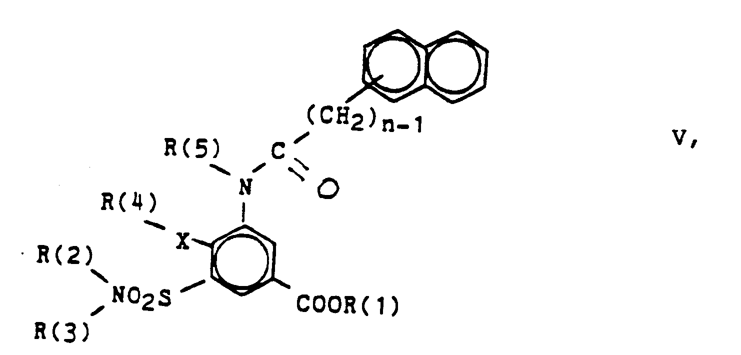 de manière connue en elle-même, en composés I,
ou en ce que l'on fait réagir :
b) des composés II 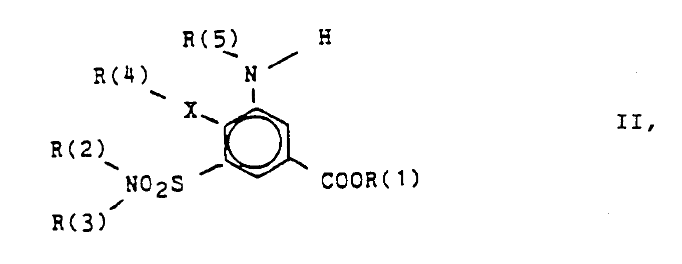 de manière connue en elle-même, avec des composés IV

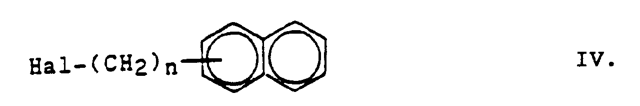

4. Médicament pour traiter l'anémie à cellules incurvées, constitué d'une quantité efficace d'un composé I conforme à la revendication 1 et des additifs habituels.

5. Utilisation d'un composé I conforme à la revendication 1 pour préparer un médicament destiné au traitement de l'anémie à cellules incurvées.

6. Composé I conforme à la revendication 1, à utiliser dans un procédé pour traiter l'anémie à cellules incurvées.
